Europäisches Patentamt

European Patent Office

Office européen des brevets

(19)

(11) Veröffentlichungsnummer: **0 354 507 B1**

(12) **EUROPÄISCHE PATENTSCHRIFT**

(45) Veröffentlichungstag der Patentschrift: **06.10.93**

(21) Anmeldenummer: **89114522.9**

(22) Anmeldetag: **07.08.89**

(51) Int. Cl.5: **C07K 1/12**, C07K 7/40, //C07K99:26

(54) Verfahren zur Herstellung von Des-B30-Insulinen und Des-B30-Insulinderivaten.

(30) Priorität: **10.08.88 DE 3827121**

(43) Veröffentlichungstag der Anmeldung:
**14.02.90 Patentblatt 90/07**

(45) Bekanntmachung des Hinweises auf die
Patenterteilung:
**06.10.93 Patentblatt 93/40**

(84) Benannte Vertragsstaaten:
**AT BE DE ES FR GB GR IT LU NL**

(56) Entgegenhaltungen:
**SU-A- 469 686**

**NATURE, Band 280, 1979, London (GB); K. MORIHARA et al., Seite 412-413&NUM;**

(73) Patentinhaber: **HOECHST AKTIENGESELL-SCHAFT**

**D-65926 Frankfurt(DE)**

(72) Erfinder: **Grünwald, Wolfgang**
**Danziger Allee 110b**
**D-6203 Hochheim(DE)**
Erfinder: **Müller-Lehar, Jürgen, Dr.**
**75, Hatheway Drive**
**West Warwick RI 02893(US)**
Erfinder: **Worm, Manfred, Dr.**
**Erbsenacker 27**
**D-6200 Wiesbaden(DE)**

EP 0 354 507 B1

**Beschreibung**

Für die Behandlung des Diabetes mellitus werden erhebliche Mengen Humaninsulin benötigt.

Die industrielle Gewinnung von Humaninsulin erfolgt überwiegend nach semisynthetischen Verfahren, welche von Schweineinsulin ausgehen. Schweineinsulin unterscheidet sich von Humaninsulin lediglich durch eine einzige Aminosäure am C-terminalen Ende der Insulin-B-Kette; im Schweineinsulin ist die B30-Position durch die Aminosäure Alanin (Ala), im Humaninsulin durch die Aminosäure Threonin (Thr) - jeweils in der L-Form - besetzt.

Zu Humaninsulin kommt man ausgehend von Schweineinsulin somit durch den Austausch von B30-Ala durch Thr. Dieser Austausch wird im wesentlichen nach zwei Verfahrenskategorien durchgeführt: den "Eintopf-Verfahren" und den "Zweistufen-Verfahren".

Nach den "Eintopf-Verfahren" wird der B30-Ala-Rest des Schweineinsulins in einem einzigen Verfahrensschritt gegen den Thr-Rest ausgetauscht (Transpeptidierung).

Nach den "Zweistufen-Verfahren" wird in einer ersten Verfahrensstufe der B30-Ala-Rest abgespalten (Proteolyse) und dann in einer separaten zweiten Verfahrensstufe ein Thr-Derivat angeknüpft (Kupplungsreaktion).

Sowohl bei dem "Eintopf-" als auch bei dem "Zweistufen"-Verfahren wird die Aminosäure Thr üblicherweise in Form eines Derivats (mit Schutzgruppen eingesetzt, so daß nach der Transpeptidierung bzw. Kupplung eine Abspaltung der Schutzgruppen erforderlich ist.

Das in der ersten Stufe der "Zweistufen-Verfahren" aus dem Schweineinsulin gebildete Des-B30-Ala-Schweineinsulin unterscheidet sich hinsichtlich seiner biologischen Aktivität von Humaninsulin nur unwesentlich. Diese erste Verfahrensstufe wird üblicherweise in Gegenwart von Enzymen, z.B. von Carboxypeptidase A, durchgeführt. Die Enzyme bewirken jedoch nicht nur die (erwünschte) Abspaltung des B30-Ala, sondern auch die (unerwünschte) Abspaltung des C-terminalen Asparagins der A-Kette (A21-Asn). Es ist daher erforderlich, die Reaktionsbedingungen so zu wählen, daß B30-Ala möglichst vollständig abgespalten und A21-Asn möglichst nicht angegriffen wird. Dieses Ziel soll nach E.W. Schmitt und H.G. Gattner, Hoppe-Seyler's Z.Physiol.Chem. Band 359, S. 799-802 (1978), hauptsächlich durch die Anwesenheit von $NH_4^+$-Ionen erreicht werden. Gemäß der in dieser Literaturstelle enthaltenen Verfahrensvorschrift wird in wässriger Lösung im alkalischen Bereich - speziell bei pH 8,3 - und einer niedrigen Substratkonzentration - speziell 5 mg Schweineinsulin/ml - gearbeitet.

Als Enzym wird Carboxypeptidase A verwendet; das Enzym/Substrat-Verhältnis beträgt 1 : 100 (Gewichtsteile).

Die mit der B30-Ala-Abspaltung konkurrierende A21-Asn-Abspaltung wird jedoch offensichtlich auch durch die Gegenwart von $NH_4^+$-Ionen nicht vollständig bzw. jedenfalls nicht ganz in dem gewünschten Maß unterdrückt.

Dies geht z.B. aus der EP-A-0017938 hervor; darin sind einige Beispiele enthalten, in denen nach der Vorschrift von E.W. Schmitt und H.G. Gattner a.a.O. gearbeitet wurde und bei denen entweder ein unklares Resultat oder noch ein erheblicher Anteil an Des-A21-Asn-Produkt erhalten wurde.

Nach Beispiel 1 (auf S. 12/13) wurde Schweineinsulin (500 mg) in wäßriger Lösung bei pH 8,3 in Gegenwart von $NH_4HCO_3$ bei einer Substratkonzentration von 5 mg/ml (Enzym:Carboxypeptidase A) bis zu einem Umsatz von 77 % reagieren gelassen. Als Ausbeute werden 460 mg = 92 % angegeben.

Wenn die Reaktion nur bis zu einem Umsatz von 77 % geführt wurde, kann jedoch rein theoretisch nicht mehr als höchstens nur etwa 385 mg (= 77 % der eingesetzten Schweineinsulin-Menge) Des-B30-Ala-Produkt entstehen. Das Beispiel ist somit nicht aussagekräftig und läßt keinen Schluß über die tatsächliche Unterdrückung der A21-Asn-Abspaltung zu.

Aussagekräftiger ist dagegen das Beispiel gemäß dem ersten Absatz auf S. 16 der EP-A.

Gemäß diesem Beispiel wurden 200 mg Rinderinsulin in einem wäßrigen $NH_4HCO_3$-Puffer bei einer Substratkonzentration von 5 mg/ml mit Carboxypeptidase A inkubiert und über Nacht stehen gelassen. Dabei sollen 180 mg eines Produkts erhalten worden sein, welches zu 78 % aus Des-B30-Ala-Rinderinsulin und 22 % Des-B30-Ala-Des-A21-Asn-Rinderinsulin bestand.

Rinder- und Schweineinsulin unterscheiden sich lediglich in den Positionen 8 und 10 der A-Kette. Es ist daher davon auszugehen, daß bezüglich der Abspaltung von B30-Ala und A21-Asn zwischen Rinder- und Schweineinsulin kein Unterschied besteht.

Bei der eigenen Nacharbeitung der Vorschrift von E.W. Schmitt und H.G. Gattner zur Herstellung von Des-B30-Ala-Schweineinsulin wurde ein Des-B30-Ala-Des-A21-Asn-Schweineinsulin-Anteil in der gemäß der vorerwähnten EP-A erwarteten Größenordnung (16 %) erhalten.

In dem Bestreben, die bekannten Verfahren zur B30-Ala-Abspaltung von Schweineinsulin zu verbessern und dabei insbesondere die mit der B30-Ala-Abspaltung konkurrierende A21-Asn-Abspaltung weiter zu unterdrücken, wurde nun gefunden, daß dies durch ein Arbeiten in Abwesenheit von $NH_4^+$-Ionen sowie bei höheren als den bisher üblichen Substrat-Konzentrationen - d.h. oberhalb von 5 mg/ml - gelingt; das Verfahren funktioniert außerdem nicht nur bei Schweineinsulin, sondern auch bei anderen Insulinen und auch Insulinderivaten sowie natürlichen und unnatürlichen Insulinvorstufen, bei denen der ganze auf B29 folgende Peptidrest ("B30-Peptidrest") abgespalten wird.

Durch das Verfahren können bei vollständigem Substratumsatz praktisch 100%ige B30-Aminosäure- bzw. B30-Peptidrest-Abspaltungsraten bei nur minimaler A21-Asn-Abspaltung erzielt werden. Aus diesem Grund und auch wegen der höheren Substratkonzentrationen sind die Raum/Zeit-Ausbeuten gegenüber den bekannten Verfahren beträchtlich erhöht. Schließlich wird auch die bei den bekannten - in Gegenwart speziell von $NH_4HCO_3$ arbeitenden - Verfahren oft auftretende sehr unangenehme Schaumbildung, die die Neigung der Insuline zum Denaturieren verstärkt, vermieden.

Das Gelingen und der Fortschritt des Verfahrens sind sehr überraschend, weil man aufgrund der Literaturstelle von E.W. Schmitt und H.G. Gattner a.a.O. nicht erwarten konnte, daß in Abwesenheit von $NH_4^+$-Ionen eine Verfahrensverbesserung erreichbar ist. Offenbar kommt die Verfahrensverbesserung hier durch die Abwesenheit von $NH_4^+$-Ionen in Kombination mit der - im Vergleich zum Stand der Technik - höheren Substratkonzentration zustande.

Erfindungsgegenstand ist ein Verfahren zur Herstellung von Des-B30-Insulinen und Des-B30-Insulinderivaten durch enzymatische Abspaltung der B30-Aminosäure aus Insulinen oder Insulinderivaten sowie des B30-Peptidrests aus natürlichen und unnatürlichen Insulinvorstufen in wäßriger Lösung bei pH-Werten oberhalb 7 (d. h. im alkalischen Bereich), das dadurch gekennzeichnet ist, daß man die enzymatische Abspaltung in Abwesenheit von $NH_4^+$-Ionen und bei einer Konzentration der Insulin-Ausgangsmaterialien von oberhalb 5 mg/ml durchführt.

Als Insulin-Ausgangsmaterialien kommen für das erfindungsgemäße Verfahren im Prinzip alle möglichen tierischen Insuline sowie auch Humaninsulin in Frage, wenngleich der Einsatz des letzteren hier wenig sinnvoll ist. Beispielhafte tierische Insuline sind Schweine-, Rinder-, Hunde-, Spermwal-, Finnwal-, Schaf-, Pferde und Kaninchen-Insulin. Bevorzugte tierische Insuline sind diejenigen mit der A1-A21 sowie B1-B29-Sequenz des Humaninsulins (Schweine-, Hunde-, Spermwal-, Finnwal- und Kaninchen-Insulin); besonders bevorzugtes Ausgangsprodukt ist Schweineinsulin.

Die Insuline können auch in Form ihrer Derivate eingesetzt werden; als solche kommen in Frage die Salze, Komplexe, mit Schutzgruppen versehene Insuline, Insuline mit am N-terminalen Ende der B-Kette oder auch der A-Kette abgespaltenen Aminosäuren (z.B. Des-Phe-B1-Schweineinsulin, Des-Gly-A1-Schweineinsulin) etc.

Auch natürliche und unnatürliche Insulinvorstufen, bei denen sich an B29 noch ein Peptidrest aus 2 oder mehreren Aminosäuren anschließt, können als Insulin-Ausgangsmaterialien verwendet werden, wobei auch hier die Produkte mit der A1-A21 sowie B1-B29-Sequenz des Humaninsulins bevorzugt sind. Beispielhafte natürliche und unnatürliche Insulinvorstufen sind Schweineproinsulin, Schweinepräproinsulin, Affenpräproinsuline und deren gentechnologisch abgewandelten Varianten, sowie B31-Arg-Insulin und B31-B32-Di-Arg-Insulin.

Die für das Verfahren einsetzbaren Enzyme sind die für derartige B30-Aminosäure-Abspaltungen bekannten Carboxypeptidasen und Proteasen. Bevorzugt sind Carboxypeptidase A, Lysylendopeptidase und Achromobacter-Protease. Die Enzyme können alleine oder in Mischung miteinander und sowohl frei als auch an einem Träger fixiert eingesetzt werden.

Das Verhältnis von eingesetzter Enzymaktivität zu Insulin-Ausgangsmaterialien kann in weiten Grenzen schwanken. Bevorzugt ist ein Verhältnis zwischen etwa 0,001 und 1 U (= Units)/mg, insbesondere zwischen etwa 0,005 und 0,2 U/mg. Die Enzymaktivität wird nach bekannten Methoden bestimmt, z.B. von Carboxypeptidase A mit N-Benzoylglycyl-L-Phenylalanin, und von Lysylendopeptidase mit Acetyllysinmethylester.

Die Reaktion wird in wäßrigem Medium bei pH-Werten oberhalb 7 - also im alkalischen Bereich - durchgeführt. Die obere pH-Grenze liegt bei etwa 10. Der bevorzugte pH-Bereich liegt bei etwa 8 - 9,5, insbesondere bei etwa 9 - 9,5.

Die Einstellung des pH-Wertes der wäßrigen Lösung bzw. Suspension kann durch Zugabe von anorganischen und/oder organischen Basen wie z.B. NaOH, KOH, $(C_2H_5)_3N$, $n\text{-}C_4H_9NH_2$, N-Ethylmorpholin, etc. erfolgen.

Die Konzentration der Insulin-Ausgangsmaterialien muß oberhalb 5 mg/ml Reaktionsmedium liegen. Die obere Grenze der Substratkonzentration beträgt etwa 500 mg/ml. Die bevorzugten Substratkonzentrationen liegen zwischen etwa 70 und 250 mg/ml, insbesondere zwischen etwa 80 und 120 mg/ml. Bei den höheren Konzentrationen ist auch ein etwas höherer pH-Wert einzustellen, da sich die Insulin-Ausgangsmaterialien bei den niedrigeren pH-Werten schlechter lösen.

Die Reaktionstemperatur liegt normalerweise zwischen etwa 0 und 50°C, vorzugsweise zwischen etwa 20 und 30°C,
die Reaktionszeit im allgemeinen zwischen etwa 5 und 24 Stunden, je nach Enzymmenge und Reaktionstemperatur.

Aus den Ausgangs-Insulinen entstehen nach dem erfindungsgemäßen Verfahren in hohen Ausbeuten die entsprechenden Des-B30-Insuline, und aus den Ausgangs-Insulinderivaten die entsprechenden Des-B30-Verbindungen. Letztere können nach üblichen Verfahren gewünschtenfalls noch in die jeweiligen Des-B30-Insuline überführt werden. Aus den natürlichen und unnatürlichen Insulinvorstufen entstehen durch Abspaltung des ganzen B30-Peptidrests ebenfalls die entsprechenden Des-B30-Insuline.

Die Endprodukt-Isolierung erfolgt bevorzugt durch Fällung am isoelektrischen Punkt oder als Zinksalz.

Die Des-B30-Insuline und -Insulinderivate können z.T. als solche für die Behandlung von Diabetes mellitus verwendet oder auch für die 2. Stufe der eingangs erwähnten "Zweistufen-Verfahrens" eingesetzt werden.

Die folgenden Vergleichsbeispiele sollen den überraschenden Charakter und den Vorteil der Erfindung gegenüber dem Stand der Technik aufzeigen; nach den Vergleichsbeispielen folgt noch eine Reihe von normalen Erfindungsbeispielen.

Vergleichsbeispiele

Die Vergleichsbeispiele wurden entsprechend sowie in geringfügiger Abwandlung der Vorschrift in der Literaturstelle E.W. Schmitt und H.G. Gattner a.a.O. durchgeführt. Die Ergebnisse sind in der nachstehenden Tabelle zusammengestellt.

Tabelle

| Versuch Nr. | Versuchsbedingungen | Ergebnis (HPLC) | | |
|---|---|---|---|---|
| | | Des-B30-Ala-SI(4) | SI(4) | Des-B30-Ala-desA21-Asn-SI(4) |
| 1 E.W.Schmitt u. H.G.Gattner a.a.O. | c(1) = 5 mg/ml, pH 8,3 (0,1m NH$_4$HCO$_3$) E/S(2) = 1 : 100, 16 Stdn., RT(3) | 84 % | – | 16 % |
| 2 Abwandlung(5) von Versuch 1 | c(1) = 5 mg/ml, pH 8,3 (NaOH), E/S(2) = 1 : 100, 16 Stdn., RT(3) | 54 % | – | 46 % |
| 3 Abwandlung(5) von Versuch 1 | c(1) = 5 mg/ml, pH 9,3 (NaOH), E/S(2) = 1 : 100, 16 Stdn., RT(3) | 23 % | – | 77 % |
| 4 Erfindungs-gemäß | c(1) = 100 mg/ml, pH 9,3 (NaOH) E/S(2) = 0,5 : 100, 15 Stdn., RT(3) | 97 % | – | 3 % |

(1) = Konzentration an Schweineinsulin

(2) = Enzym (Carboxypeptidase A)/Substrat (Schweineinsulin)-Gewichtsverhältnis, entsprechend einem Verhältnis Enzymaktivität : Schweineinsulin = Schweineinsulin = bzw.

(3) = Raumtemperatur

(4) = SI = Schweineinsulin

(5) = Die Abwandlungen gegenüber Versuch 1 sind unterstrichen

In Versuch 1 wurde die Vorschrift von E.W. Schmitt und H.G. Gattner nachgearbeitet.

In Versuch 2 wurde anstelle des NH$_4$HCO$_3$ so viel NaOH verwendet, daß gerade der gleiche pH-Wert wie in Versuch 1 (= 8,3) erreicht wurde. Es wurde ein erheblich höherer Anteil an Des-A21-Asn-Produkt erhalten.

5

In Versuch 3 wurde ebenfalls mit NaOH, jedoch bei einem etwas höheren pH-Wert gearbeitet. Das Resultat ist nochmals erheblich ungünstiger.

In dem erfindungsgemäßen Versuch 4 wurde das optimale Resultat erzielt.

## Erfindungsbeispiele

### Beispiel 1

5 g Schweineinsulin werden in 60 ml Wasser suspendiert und durch Zugabe von 1 N Natronlauge bei pH 9,2 in Lösung gebracht. Nach Zugabe von 550 U Carboxypeptidase A wird 15 Stunden bei 23°C inkubiert. Die Reaktionsmischung wird auf 5°C abgekühlt und durch Zugabe von konz. Essigsäure auf pH 3,8 gestellt. Nach Zugabe von 1 g Zinkacetat in 12,5 ml Wasser wird bei pH 5,7 gefällt. Nach HPLC enthält das isolierte Produkt 96,5 % Des-B30-Insulin.

### Beispiel 2

1 g Schweineinsulin wird nach Suspendieren in 4 ml Wasser durch Zugabe von 33 %iger Natronlauge in Lösung gebracht. Bei pH 9,2 werden 50 $\mu$l Suspension von Carboxypeptidase A zugegeben und bei 22°C inkubiert. Nach 24 Stunden zeigt die HPLC vollständige Umsetzung an. Nach Verdünnen mit 5 ml Wasser wird mit konz. Essigsäure auf pH 3,6 gestellt, 20 ml Ethanol und anschließend 385 mg Zinkacetat, gelöst in 3 ml Wasser, zugegeben. Mit 25 %iger Ammoniaklösung wird bei pH 6,0 gefällt, filtriert und getrocknet. Die Umwandlung in Des-B30-Insulin betrug 98 %.

### Beispiel 3

1 g Schweineinsulin-Zinksalz wird in 10 ml Wasser suspendiert und durch Einstellen mit 33 %iger Natronlauge auf pH 9,3 in Lösung gebracht. Nach Zugabe von 10 mg EDTA (= Ethylendiamintetraessigsäure) wird die Lösung klar. Durch Einrühren von 50 $\mu$l Carboxypeptidase-A-Suspension bei 22°C wird die Reaktion gestartet. Nach HPLC-Kontrolle wird die Reaktion am Umsatzoptimum abgebrochen und das Produkt wie in Beispiel 2 isoliert. Anstelle von Ethanol wird Methanol eingesetzt. Die Umwandlung in Des-B30-Insulin betrug nach HPLC 99 %.

### Beispiel 4

1 g Schweineinsulin wird analog Beispiel 1 unter Einsatz von Kalilauge umgesetzt. Nach HPLC betrug die Umwandlung in Des-B30-Insulin 98,5 %.

### Beispiel 5

1 g Schweineinsulin wird in 4 ml Wasser gelöst und mit konz. Natronlauge bei pH 9,0 gelöst. Anschließend wird die Reaktion bei 22°C durch Zugabe von 20 U (ALM = Acetyllysylmethylester) Lysylendopeptidase gestartet. Nach 23 Stunden ergibt die Alaninbestimmung 94 % Abspaltung. Es wird mit 5 ml Wasser verdünnt und durch Ansäuern mit Essigsäure bei pH 5,4 gefällt, isoliert und getrocknet.

### Beispiel 6

1 g Schweineinsulin wird in 4 ml Wasser suspendiert und durch Zugabe von N-Ethylmorpholin bei pH 9,5 gelöst. Die weitere Durchführung erfolgt wie in Beispiel 5 beschrieben. Die Umwandlung in Des-B30-Insulin betrug 98 %.

### Beispiel 7

1 g Schweineinsulin wird nach Suspendieren in 12 ml Wasser durch Zugabe von 33 %iger Natronlauge in Lösung gebracht. Bei pH 9,3 werden 20 U (ALM) Lysylendopeptidase, fixiert auf einem neutralen Träger, zugegeben und 24 Std. bei 23°C stehen gelassen. Der Umsatz nach HPLC beträgt 92 %.

Beispiel 8

1 g Schweineinsulin-Zinksalz wird in 12 ml Wasser suspendiert und durch Einstellen von pH 9,3 mit Triethylamin unter Zusatz von 50 mg EDTA in Lösung gebracht. Durch Zugabe von 110 U Carboxypeptidase A wird die Reaktion gestartet. Nach 15 Stdn. betrug die Umsetzung zu Des-B30-Insulin 94,6 %.

**Patentansprüche**

1. Verfahren zur Herstellung von Des-B30-Insulinen und Des-B30-Insulinderivaten durch enzymatische Abspaltung der B30-Aminosäure aus Insulinen oder Insulinderivaten sowie des B30-Peptidrests aus natürlichen und unnatürlichen Insulinvorstufen in wäßriger Lösung bei pH-Werten oberhalb 7, dadurch gekennzeichnet, daß man die enzymatische Abspaltung in Abwesenheit von $NH_4^+$-Ionen und bei einer Konzentration der Insulin-Ausgangsmaterialien von oberhalb 5 mg/ml Reaktionsmischung durchführt.

2. Verfahren nach Anspruch 1, dadurch gekennzeichnet, daß man von Insulinen oder Insulinderivaten sowie natürlichen oder unnatürlichen Insulinvorstufen mit der A1-A21-und B1-B29-Sequenz des Humaninsulins ausgeht.

3. Verfahren nach Anspruch 1 oder 2, dadurch gekennzeichnet, daß man von Schweineinsulin ausgeht.

4. Verfahren nach einem oder mehreren der Ansprüche 1 bis 3, dadurch gekennzeichnet, daß man als Enzyme für die enzymatische Abspaltung Carboxypeptidase A, Lysylendopeptidase, Achromobacter-Protease und/oder Trypsin verwendet.

5. Verfahren nach einem oder mehreren der Ansprüche 1 bis 4, dadurch gekennzeichnet, daß ein Verhältnis von Enzymaktivität zu Insulin-Ausgangsmaterial zwischen etwa 0,001 und 1 U/mg, vorzugsweise zwischen etwa 0,005 und 0,2 U/mg, eingestellt ist.

6. Verfahren nach einem oder mehreren der Ansprüche 1 bis 5, dadurch gekennzeichnet, daß die Konzentration der Insulin-Ausgangsmaterialien zwischen etwa 70 und 250 mg/ml, insbesondere zwischen etwa 80 und 120 mg/ml Reaktionsmischung liegt.

**Claims**

1. A process for the preparation of des-B30-insulins and des-B30-insulin derivatives by enzymatic elimination of the B30 amino acid from insulins or insulin derivatives as well as of the B30 peptide residue from natural and unnatural insulin precursors in aqueous solution at pH values above 7, which comprises carrying out the enzymatic elimination in the absence of $NH_4^+$ ions and with a concentration of the insulin starting materials above 5 mg/ml of reaction mixture.

2. The process as claimed in claim 1, which starts from insulins or insulin derivatives as well as natural or unnatural insulin precursors having the A1-A21 and B1-B29 sequence of human insulin.

3. The process as claimed in claim 1 or 2, which starts from porcine insulin.

4. The process as claimed in one or more of claims 1 to 3, wherein the enzymes used for the enzymatic elimination are carboxypeptidase A, lysyl endopeptidase, Achromobacter protease and/or trypsin.

5. The process as claimed in one or more of claims 1 to 4, wherein the ratio of enzyme activity to insulin starting material is adjusted to between about 0.001 and 1 U/mg, preferably between about 0.005 and 0.2 U/mg.

6. The process as claimed in one or more of claims 1 to 5, wherein the concentration of the insulin starting materials is between about 70 and 250 mg/ml, in particular between about 80 and 120 mg/ml, of reaction mixture.

**Revendications**

1. Procédé de fabrication de des-B30-insulines et de dérivés de des-B30-insulines, par coupure enzymatique de l'acide aminé B30 des insulines ou dérivés d'insuline ainsi que du peptide B30 restant provenant de précurseurs naturels et non naturels d'insuline, en solution aqueuse à pH supérieur à 7, caractérisé en ce qu'on réalise la coupure enzymatique en l'absence d'ions $NH_4^+$ et à une concentration des matières premières insuliniques de plus de 5 mg/ml de mélange réactionnel.

2. Procédé selon la revendication 1, caractérisé en ce qu'on part d'insuline ou de dérivés d'insuline ainsi que de précurseurs naturels et non naturels d'insuline présentant la séquence A1-A21 et la séquence B1-B29 de l'insuline humaine.

3. Procédé selon la revendication 1 ou 2, caractérisé en ce qu'on part d'insuline de porc.

4. Procédé selon une ou plusieurs des revendications 1 à 3, caractérisé en ce qu'on utilise, comme enzyme pour la coupure enzymatique, la carboxypeptidase A, la lysylendopeptidase, l'Achromobacter-protéase et/ou la trypsine.

5. Procédé selon une ou plusieurs des revendications 1 à 4, caractérisé en ce que le rapport de l'activité enzymatique à la matière première insulinique est fixé à environ 0,001 à 1 U/mg, de préférence environ 0,005 à 0,2 U/mg.

6. Procédé selon une ou plusieurs des revendications 1 à 5, caractérisé en ce que la concentration des matières premières insuliniques est comprise entre environ 70 et 250 mg/ml, en particulier entre environ 80 et 120 mg/ml de mélange réactionnel.